(19)

**Europäisches Patentamt**
**European Patent Office**
**Office européen des brevets**

(11) **EP 4 537 835 A1**

(12) **EUROPEAN PATENT APPLICATION**

(43) Date of publication:
16.04.2025 Bulletin 2025/16

(21) Application number: 24206491.3

(22) Date of filing: 14.10.2024

(51) International Patent Classification (IPC):
$A61K\ 35/742^{(2015.01)}$    $A61K\ 31/00^{(2006.01)}$
$A61K\ 31/19^{(2006.01)}$    $A61K\ 31/195^{(2006.01)}$
$A61P\ 15/08^{(2006.01)}$

(52) Cooperative Patent Classification (CPC):
A61K 35/742; A61K 31/00; A61K 31/19;
A61K 31/195; A61P 15/08

(84) Designated Contracting States:
AL AT BE BG CH CY CZ DE DK EE ES FI FR GB
GR HR HU IE IS IT LI LT LU LV MC ME MK MT NL
NO PL PT RO RS SE SI SK SM TR
Designated Extension States:
BA
Designated Validation States:
GE KH MA MD TN

(30) Priority: 12.10.2023 IT 202300021306

(83) Declaration under Rule 32(1) EPC (expert solution)

(71) Applicant: Nutra Futura S.r.l.
20155 Milano (IT)

(72) Inventor: UBERTI, Francesca
20155 Milano (IT)

(74) Representative: Marben S.r.l.
Via Larga, 16
20122 Milano (IT)

(54) **MIXTURE, COMPOSITIONS CONTAINING THAT MIXTURE AND USE THEREOF IN THE TREATMENT OF FEMAL INFERTILITY**

(57)    The present invention relates to a mixture, compositions containing that mixture, and the use thereof in the treatment of female infertility, particularly to promote and enhance female gametogenesis.

In greater detail, the present invention relates to a mixture comprising or, alternatively, consisting of: at least one strain of bacteria belonging to the species *Lactobacillus crispatus,* N-acetyl-cysteine (NAC) and lycopene.

Furthermore, the present invention relates to a mixture comprising or, alternatively, consisting of: at least one strain of bacteria belonging to the species *Lactobacillus crispatus,* N-acetyl-cysteine (NAC) and lycopene and, optionally, one or more of: vitamin 0, preferably vitamin D3, folic acid and/or mixtures thereof.

Furthermore, the present invention relates to compositions comprising said mixture.

Furthermore, the present invention relates to said mixture and/or compositions comprising it for use in the treatment, preventive and/or curative, of female infertility, particularly to promote and enhance female gametogenesis.

EP 4 537 835 A1

**Description**

The present invention relates to a mixture, compositions containing that mixture, and the use thereof in the treatment of female infertility, preferably to promote and enhance female gametogenesis.

In greater detail, the present invention relates to a mixture comprising or, alternatively, consisting of: at least one strain of bacteria belonging to the species *Lactobacillus crispatus,* N-acetyl-cysteine (NAC) and lycopene. Furthermore, the present invention relates to a mixture comprising or, alternatively, consisting of: at least one strain of bacteria belonging to the species *Lactobacillus crispatus,* N-acetyl-cysteine (NAC) and lycopene and, optionally, one or more of: vitamin D, preferably vitamin D3, folic acid and/or mixtures thereof.

Furthermore, the present invention relates to compositions comprising said mixture.

Furthermore, the present invention relates to said mixture and/or compositions comprising it for use in the treatment, preventive and/or curative, of female infertility, preferably to promote and enhance female gametogenesis.

**BACKGROUND OF THE INVENTION**

Female gametogenesis involves the natural processes of folliculogenesis, oocyte maturation, ovulation and the luteal phase.

Folliculogenesis is a natural process adapted to bring about the growth, maturation and expulsion of a mature oocyte by the follicle, a fundamental moment of a woman's fertility and of the subsequent transformation of the follicle into the corpus luteum, with the consequent production of progesterone, a hormone that is critical for implantation and the maintenance of a pregnancy in the initial phases.

All these processes are regulated by hormones (mainly FSH and LH, but also the anti-müllerian hormone, inhibin A, inhibin B, and still others) and molecules with paracrine activity which act as local/ tissue messengers.

FSH and LH, produced at the hypothalamic-pituitary level, bring about, at the ovarian level, in very precise times and manners, the development of the follicle and the events described above.

FSH and LH are produced daily at different concentrations, such as to determine very precise phases of the ovulation cycle (follicular phase: ↑FSH ↓LH; ovulatory phase: ↑FSH ↑LH: luteal phase: ↓FSH ↑LH)

Their action depends on a large number of molecules (second messengers) which transmit messages into the target cells of FSH and LH.

An alteration of the processes involved in female gametogenesis occurs, for example, in women of reproductive age suffering from ovulation disorders, for example polycystic ovary syndrome (PCOS) or genito-pelvic endometriosis, in women in whom, for example due to biological age, such physiological processes are less efficient or in women undergoing medically assisted procreation techniques, in which the hormonal treatment with gonadotropins shows to negatively alter those processes.

For women there thus exists a need to be able to have novel methods capable of controlling and improving normal ovulatory activity in different situations, both physiological and pathological, and of antagonising the negative effects caused by the use of hormones, so as to control and enhance female gametogenesis, promote the development of healthy mature gametes and increase fertility.

Furthermore, there remains a great need to provide new, effective methods for treating female infertility which do not have side effects, are well tolerated by the treated subjects and are also easily administrated.

The technical problem that the present invention addresses and resolves is thus that of providing a valid solution for effectively treating female infertility, in a preventive and/or curative manner, particularly to promote and enhance female gametogenesis, by promoting all the processes involved, namely folliculogenesis, oocyte maturation, ovulation and the luteal phase, in the absence of or with reduced side effects.

The Applicant, by virtue of an intense research activity, has developed a mixture and compositions containing it (briefly, the mixture and compositions of the invention), which are effective, devoid of side effects and with high tolerability, for use in a method of treatment, preventive and/or curative, of female infertility, particularly to promote and enhance female gametogenesis, by promoting all the processes involved, namely folliculogenesis, oocyte maturation, ovulation and the luteal phase, and thus promoting the development of healthy mature gametes.

In particular, the mixture and/or compositions containing it of the invention, thanks to the presence of natural ingredients, in safety (i.e. without side effects and/or with high tolerability), by promoting all the processes involved in female gametogenesis, namely folliculogenesis, oocyte maturation, ovulation and the luteal phase, and thus promoting the development of healthy mature gametes, are useful for treating and/or preventing female infertility, in particular for promoting and enhancing female gametogenesis.

**SUMMARY OF THE INVENTION**

In a first aspect, the present invention provides a mixture comprising or, alternatively, consisting of: i) at least one strain of bacteria belonging to the species *Lactobacillus crispatus,* ii) N-acetyl-cysteine (NAC) and iii) lycopene.

For example, the at least one strain of bacteria belonging to the species *Lactobacillus crispatus* is the strain *Lactobacillus crispatus* nova LCR deposited by Probionova SA on 26.07.2022 at the *Deutsche Sammlung von Mikroorganismen und Zellkulturen* (DSMZ) under deposit number DSM 34348; preferably, at a concentration comprised from $1{\times}10^6$ CFU to $1{\times}10^{12}$ CFU, more preferably comprised from $1{\times}10^8$ CFU to $1{\times}10^{10}$ CFU, for example $1{\times}10^9$ CFU.

For example, the at least one strain of bacteria belonging to the species *Lactobacillus crispatus* is the strain *Lactobacillus crispatus GA31* deposited by Probionova SA on 17.07.2024 at the *Deutsche Sammlung von Mikroorganismen und Zellkulturen* (DSMZ) under deposit number DSM 35105; preferably, at a concentration comprised from $1{\times}10^6$ CFU to $1{\times}10^{12}$ CFU, more preferably comprised from $1{\times}10^8$ CFU to $1{\times}10^{10}$ CFU, for example $1{\times}10^9$ CFU.

For example, the N-acetyl-cysteine (NAC) is N-acetyl-L-cysteine (CAS No. 616-91-1) in the form of a 60 mesh powder.

For example, the lycopene is from tomato extract (*Solanum lycopersicum L.*)*,* in the form of microencapsulated powder with a minimum lycopene titer of 5% by weight.

In a further aspect, the present invention provides a mixture comprising or, alternatively, consisting of: i) at least one strain of bacteria belonging to the species *Lactobacillus crispatus,* ii) N-acetyl-cysteine (NAC) and iii) lycopene and which further includes one or more of: iv) vitamin D, preferably vitamin D3; v) folic acid.

Preferably, said i) at least one strain of bacteria belonging to the species *Lactobacillus crispatus* is selected from the strain of bacteria *Lactobacillus crispatus* nova LCR DSM 34348 and the strain of bacteria *Lactobacillus crispatus* GA31 DSM 35105, or mixtures thereof; preferably, each of the mentioned strains of bacteria at a concentration comprised from $1{\times}10^6$ CFU to $1{\times}10^{12}$ CFU, more preferably comprised from $1{\times}10^8$ CFU to $1{\times}10^{10}$ CFU, for example $1{\times}10^9$ CFU.

In a further aspect, the present invention provides a composition (composition of the invention) comprising said mixture of the invention and, optionally, an acceptable pharmaceutical or food grade additive and/or excipient.

In a further aspect, the present invention provides a composition (composition of the invention) as defined above for use as a medicament.

In a further aspect, the present invention provides a mixture and/or composition containing it (mixture and/or composition of the invention) as defined above for use in the treatment, preventive and/or curative, of female infertility, particularly to promote and enhance female gametogenesis.

The Applicant has found that the mixture and/or compositions containing it (mixture and/or compositions of the invention) as defined above are effective in a method of treatment, preventive and/or curative, of female infertility, particularly to promote and enhance female gametogenesis, by promoting all the processes involved, namely folliculogenesis, oocyte maturation, ovulation and the luteal phase, and thus promoting the development of healthy mature gametes.

Said preventive and/or curative treatment activity of the mixture and/or compositions of the invention is due to the specific and innovative combination of the active components (as defined in the present detailed description that follows), which imparts to the same a synergistic action/activity capable of:

- maintaining the safety of the intestinal epithelium;
- maintaining unaltered the permeability related to the opening/closing of the tight junctions;
- increasing the ion flow of the intestinal barrier to facilitate absorption;
- stimulating cell viability and antioxidant mechanisms, thereby guaranteeing protection against oxidative stress and proper mitochondrial metabolism of ovarian cells;
- reducing the inflammatory state and apoptotic mechanisms that lead to the death of ovarian cells and the failed maturation of female gametes;
- promoting the transition of meiosis from metaphase I to anaphase I in oocytes, exercising a role of controlling ovarian function;
- maintaining proper function of the hormones responsible for gamete maturation;

demonstrating safety and biological effectiveness and thus providing a useful application for treating, in a preventive and/or curative manner, female infertility, particularly to promote and enhance female gametogenesis, by promoting all the processes involved, namely folliculogenesis, oocyte maturation, ovulation and the luteal phase, and thus promoting the development of healthy mature gametes.

Furthermore, the mixture and/or compositions containing it (mixture and/or compositions of the invention), thanks to the presence of natural ingredients, do not have any significant adverse effects, are devoid of side effects and have high tolerability, and can be administered to a wide category of subjects.

Finally, the mixture and/or the compositions of the invention are easy to prepare and economical.

These and other aims, which will become apparent from the detailed description that follows, are achieved by the mixture and/or compositions containing it (mixture and/or compositions of the invention) thanks to the technical characteristics claimed in the appended claims.

## DESCRIPTION OF THE FIGURES

Two strains of bacteria belonging to the species *Lactobacillus crispatus* were tested: the strain *Lactobacillus crispatus* nova LCR deposited by Probionova SA on 26.07.2022 at the *Deutsche Sammlung von Mikroorganismen und Zellkulturen* (DSMZ) under deposit number DSM 34348, at a concentration of $1\times10^9$ CFU, and the strain *Lactobacillus crispatus GA31* deposited by Probionova SA on 17.07.2024 at the *Deutsche Sammlung von Mikroorganismen und Zellkulturen* (DSMZ) under deposit number DSM 35105, at a concentration of $1\times10^9$ CFU.

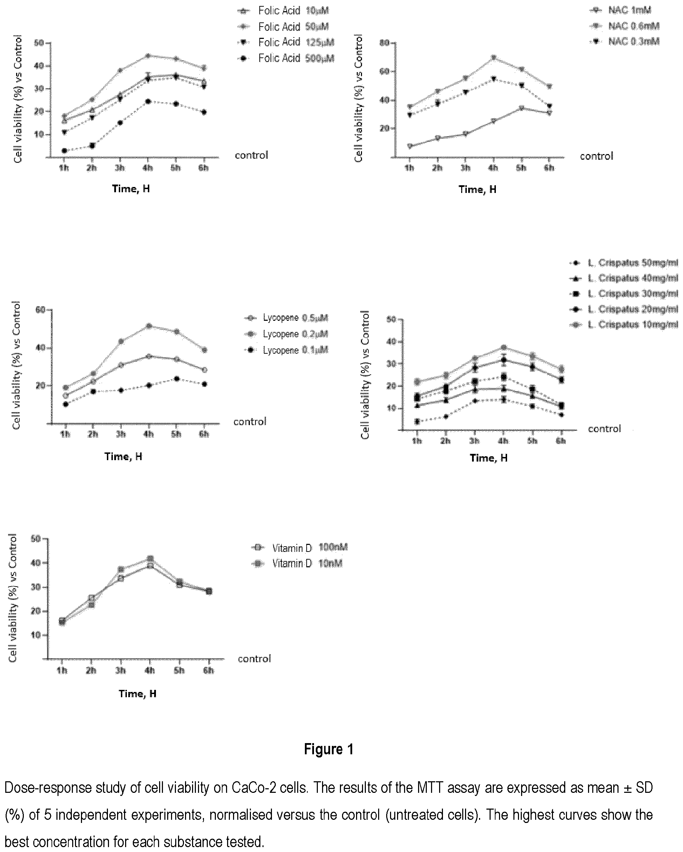

**Figure 1** shows the results of a dose-response study of cell viability on CaCo-2 cells. The results of the MTT assay are expressed as mean $\pm$ SD (%) of 5 independent experiments, normalised versus the control (untreated cells). The highest curves show the best concentration for each substance tested.

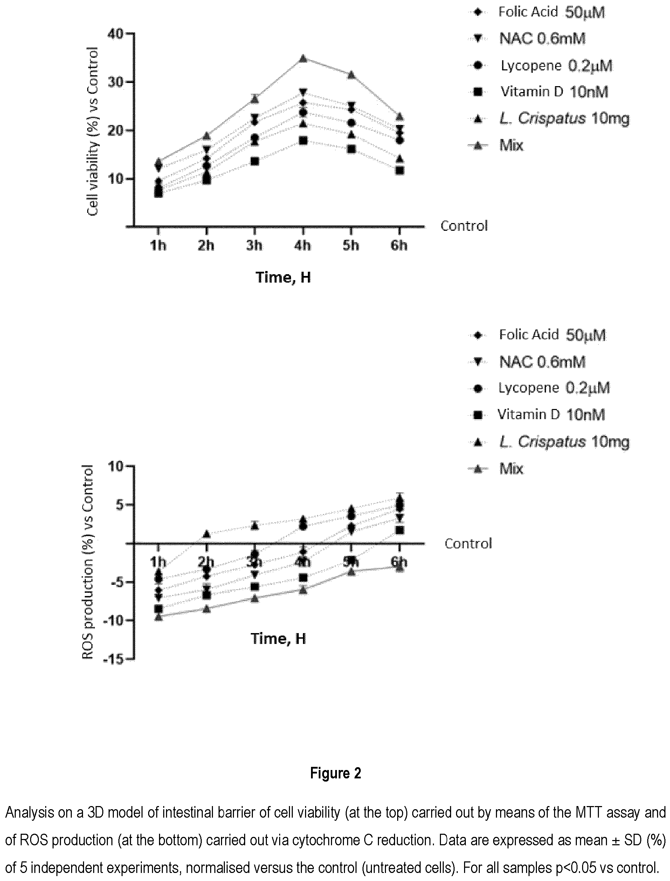

**Figure 2** shows the results of an analysis on a 3D model of the intestinal barrier of cell viability (at the top) carried out by means of the MTT assay and of ROS production (at the bottom) carried out via cytochrome C reduction. Data are expressed as mean $\pm$ SD (%) of 5 independent experiments, normalised versus the control (untreated cells). For all samples p<0.05 vs control.

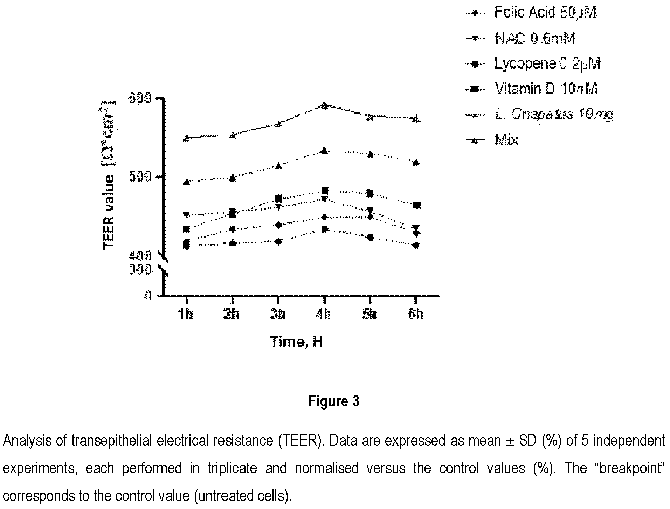

**Figure 3** shows the results of an analysis of transepithelial electrical resistance (TEER). Data are expressed as mean $\pm$ SD (%) of 5 independent experiments, each performed in triplicate and normalised versus the control values (%). The "breakpoint" corresponds to the control value (untreated cells).

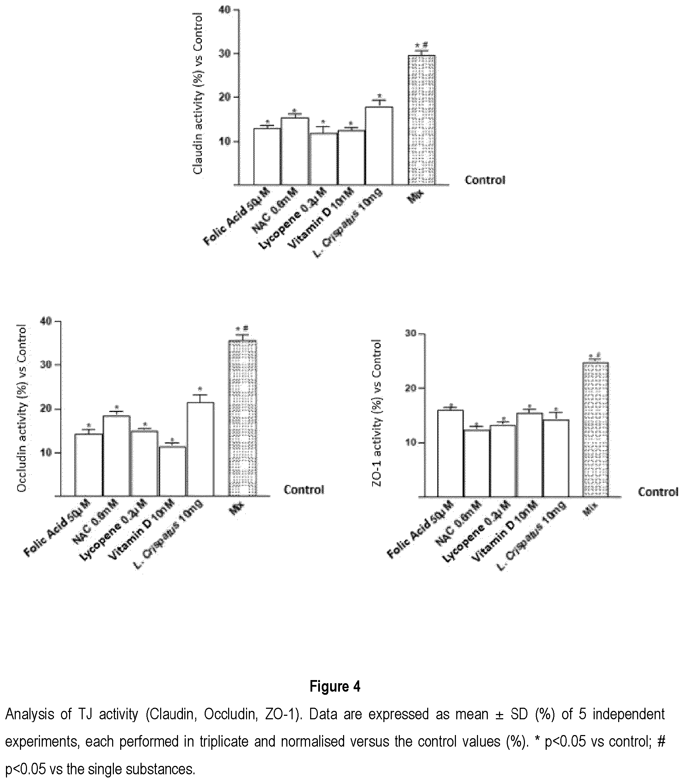

**Figure 4** shows the results of an analysis of TJ activity (claudin, occludin and ZO-1). Data are expressed as mean $\pm$ SD (%) of 5 independent experiments, each performed in triplicate and normalised versus the control values (%). * p<0.05 vs control; # p<0.05 vs the single substances.

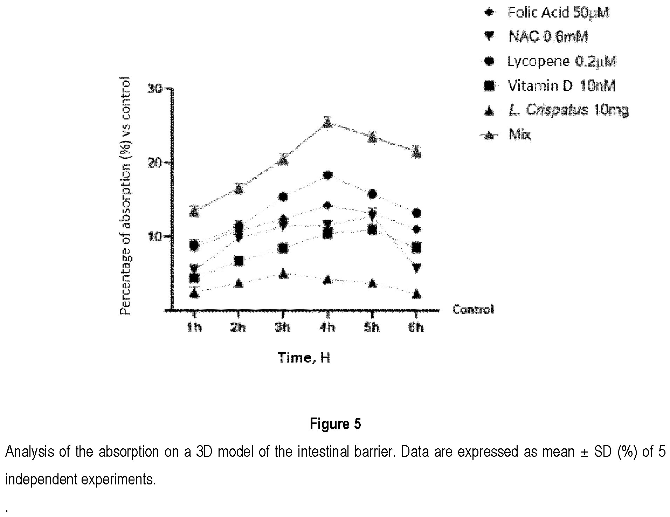

**Figure 5** shows the results of an analysis of absorption on a 3D model of the intestinal barrier. Data are expressed as mean $\pm$ SD (%) of 5 independent experiments.

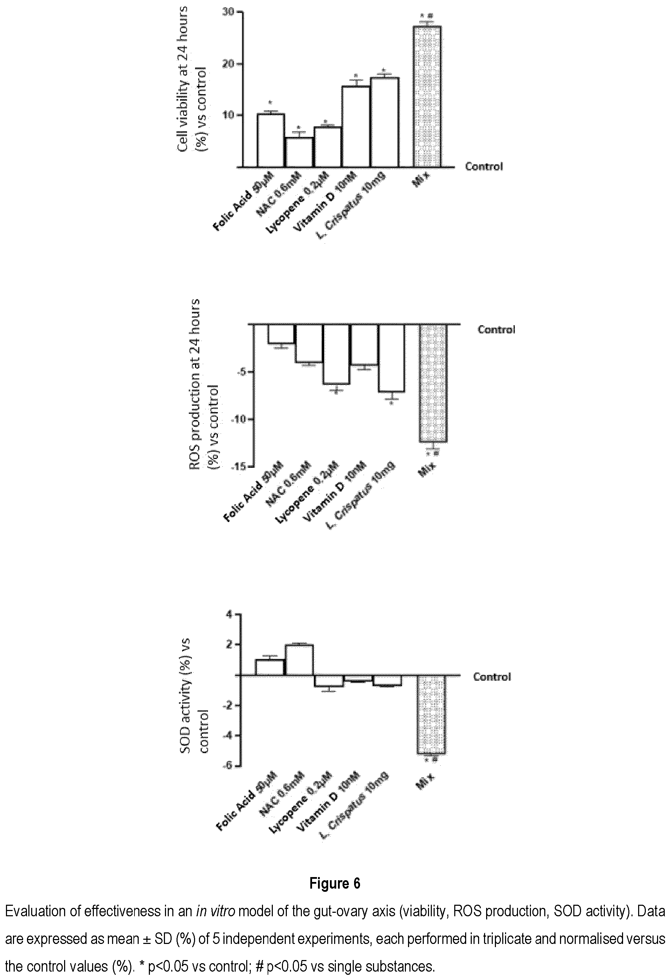

**Figure 6** shows the results of an evaluation of effectiveness in an *in vitro* model of the gut-ovary axis (viability, ROS production, SOD activity). Data are expressed as mean $\pm$ SD (%) of 5 independent experiments, each performed in triplicate and normalised versus the control values (%). * p<0.05 vs control; # p<0.05 vs single substances.

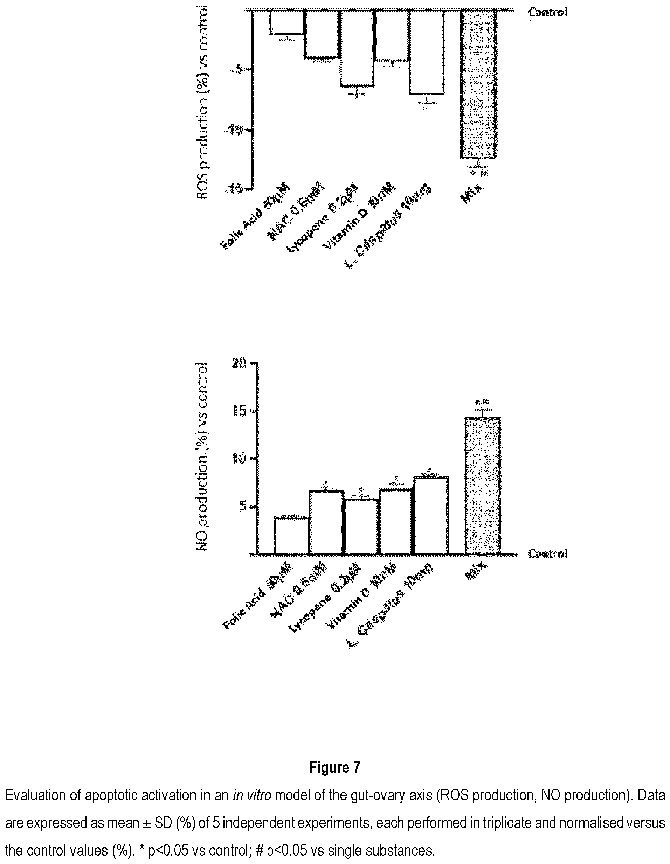

**Figure 7** shows the results of an evaluation of apoptotic activation in an *in vitro* model of the gut-ovary axis (ROS production, NO production). Data are expressed as t mean $\pm$ SD (%) of 5 independent experiments, each performed in triplicate and normalised versus the control values (%). * p<0.05 vs control; # p<0.05 vs single substances.

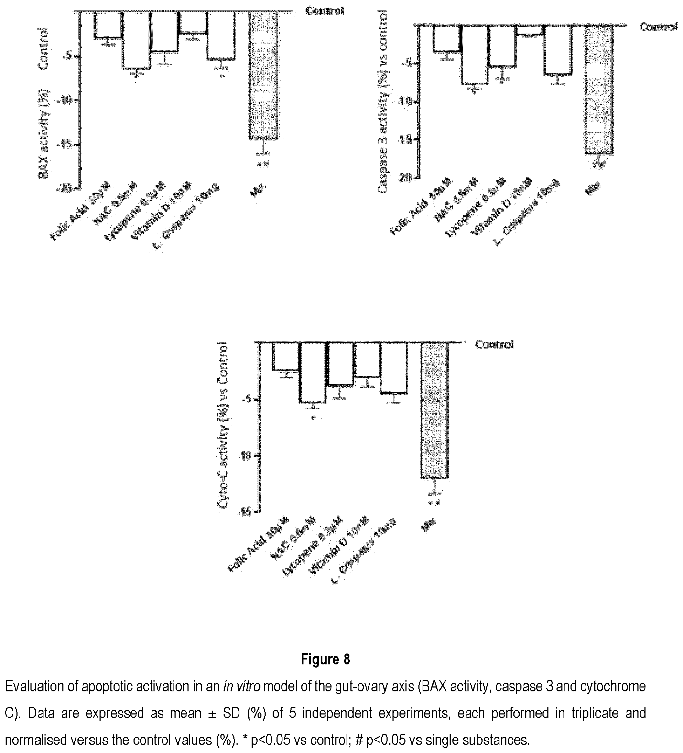

**Figure 8** shows the results of a further evaluation of apoptotic activation in an *in vitro* model of the gut-ovary axis (BAX activity, caspase 3 and cytochrome C). Data are expressed as mean $\pm$ SD (%) of 5 independent experiments, each performed in triplicate and normalised versus the control values (%). * p<0.05 vs control; # p<0.05 vs single substances.

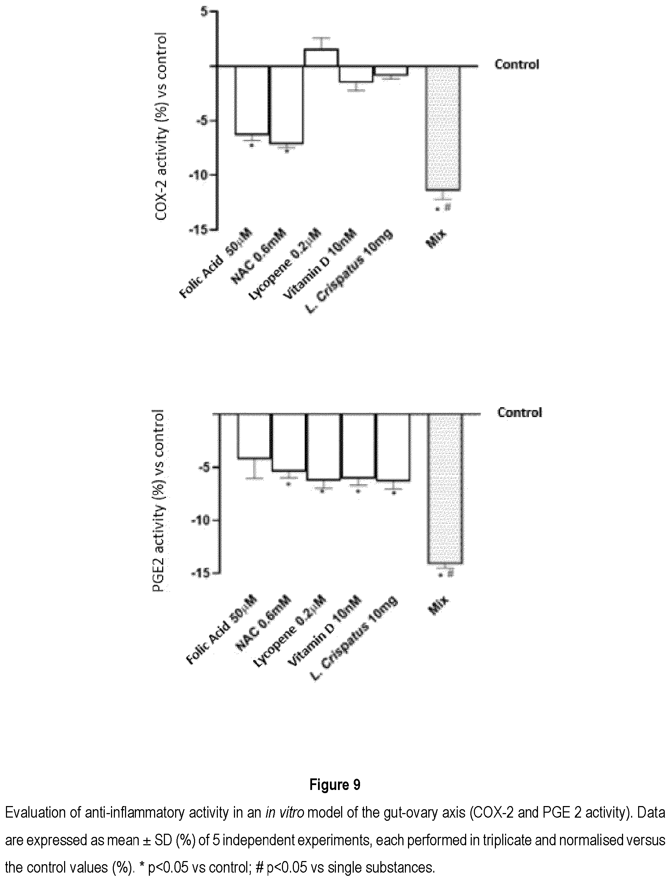

**Figure 9** shows the results of an evaluation of anti-inflammatory activity in an *in vitro* model of the gut-ovary axis (COX-2 and PGE 2 activity). Data are expressed as mean $\pm$ SD (%) of 5 independent experiments, each performed in triplicate and normalised versus the control values (%). * p<0.05 vs control; # p<0.05 vs single substances.

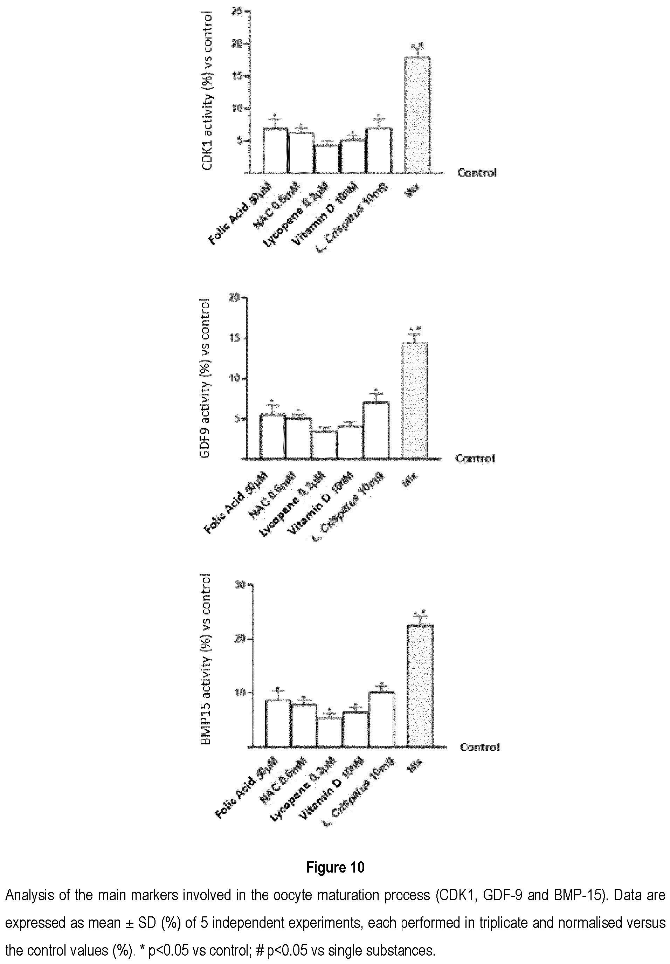

**Figure 10** shows the results of an analysis of the main markers involved in the oocyte maturation process (CDK1, GDF-9 and BMP-15). Data are expressed as mean $\pm$ SD (%) of 5 independent experiments, each performed in triplicate and normalised versus the control values (%). * p<0.05 vs control; # p<0.05 vs single substances.

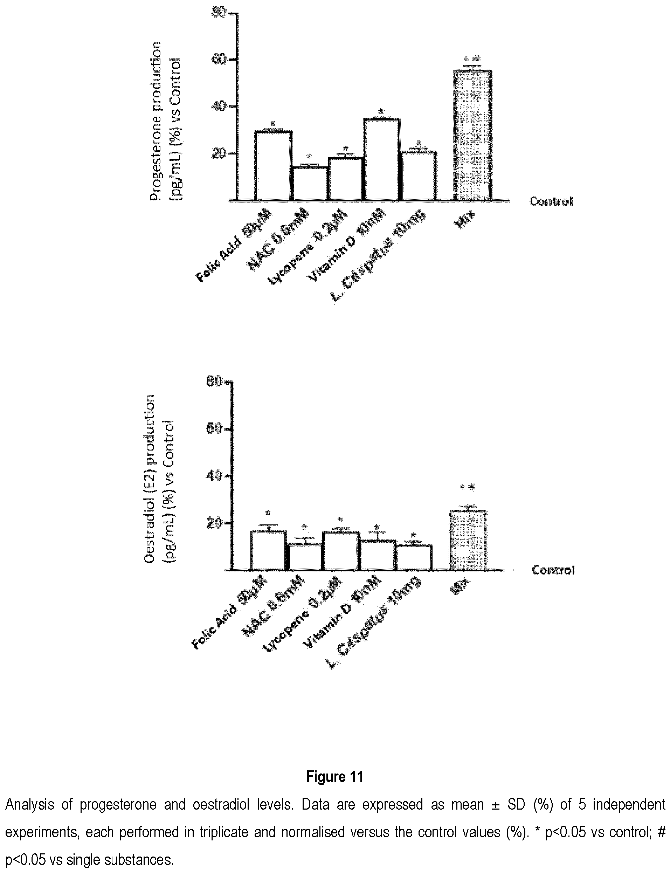

**Figure 11** shows the results of an analysis of progesterone and oestradiol levels. Data are expressed as mean $\pm$ SD (%) of 5 independent experiments, each performed in triplicate and normalised versus the control values (%). * p<0.05 vs control; # p<0.05 vs single substances.

## DETAILED DESCRIPTION OF THE INVENTION

The object of the present invention is a mixture comprising or, alternatively, consisting of:

i) at least one strain of bacteria belonging to the species *Lactobacillus crispatus;* preferably, said i) at least one strain of bacteria belonging to the species *Lactobacillus crispatus* is selected from the strain of bacteria *Lactobacillus crispatus* nova LCR DSM 34348 and the strain of bacteria *Lactobacillus crispatus* GA31 DSM 35105, or mixtures thereof; and
ii) N-acetyl-cysteine (NAC); and
iii) a lycopene.

The mixture of the present invention comprises i) at least one strain of bacteria belonging to the species *Lactobacillus crispatus.* Preferably, said i) at least one strain of bacteria belonging to the species *Lactobacillus crispatus* is selected from the strain of bacteria *Lactobacillus crispatus* nova LCR DSM 34348 and the strain of bacteria *Lactobacillus crispatus* GA31 DSM 35105, or mixtures thereof; preferably, each of the mentioned strains of bacteria at a concentration comprised from $1\times10^6$ CFU to $1\times10^{12}$ CFU, more preferably comprised from $1\times10^8$ CFU to $1\times10^{10}$ CFU, for example

$1 \times 10^9$ CFU.

*Lactobacillus Crispatus* is a common species of the genus *Lactobacillus* and is a beneficial species of the microbiota (probiotic), a producer of hydrogen peroxide ($H_2O_2$). *Lactobacillus crispatus* is a normal inhabitant of the lower reproductive tract in healthy women.

In the context of the present invention, "at least one strain of bacteria belonging to the species *Lactobacillus crispatus*" is intended to refer to, and include, any strain of *Lactobacillus crispatus* and/or mixtures of strains of *Lactobacillus crispatus,* in a viable or viable but non-replicating, non-viable, inactivated, semi-inactivated, dead but metabolically active or dead form.

In the context of the present invention, at least one strain of bacteria belonging to the species *Lactobacillus crispatus* that is "non-viable", "inactivated" or "dead" refers to a microorganism that is no longer capable, temporarily or definitively, of forming colonies in a culture. "Non-viable", "inactivated" or "dead" microorganisms can have intact or broken cells membranes. Therefore, the term "non-viable", "inactivated" or "dead" also indicates extracts and lysates of microorganisms. A particular type of "inactivated" microorganisms is "viable but non-replicating" or "dead but metabolically active" microorganisms, which are not capable of replicating, that is, of forming colonies in a culture, but retain a metabolic activity characteristic of live, non-inactivated microorganisms. The inactivated microorganisms can be produced with any method known to the person skilled in the art, such as irradiation (with gamma rays, X-rays, exposure to UV rays) or exposure to heat. The type of treatment, the intensity, the dose and the time of exposure are regulated by the person skilled in the art based on the amount and nature of the probiotic microorganisms to be inactivated.

Preferably, the at least one strain of bacteria belonging to the species *Lactobacillus crispatus* can be used in a non-viable, inactivated or dead form, preferably in a viable but non-replicating or dead but metabolically active form.

Preferably, the at least one strain of bacteria belonging to the species *Lactobacillus crispatus* is the strain *Lactobacillus crispatus* nova LCR deposited at the *Deutsche Sammlung von Mikroorganismen und Zellkulturen* (DSMZ) under deposit number DSM 34348 and/or the strain *Lactobacillus crispatus* GA31 deposited at the *Deutsche Sammlung von Mikroorganismen und Zellkulturen* (DSMZ) under deposit number DSM 35105; preferably, each of the mentioned strains of bacteria at a concentration comprised from $1 \times 10^6$ CFU to $1 \times 10^{12}$ CFU, more preferably comprised from $1 \times 10^8$ CFU to $1 \times 10^{10}$ CFU, for example $1 \times 10^9$ CFU.

The at least one strain of bacteria belonging to the species *Lactobacillus crispatus* is present in the mixture of the invention in an amount by weight, with respect to the total weight of the mixture, preferably comprised in the range from 0.5% to 2.5%, more preferably comprised from 1% to 2%, even more preferably comprised from 1.3% to 1.9%, e.g., equal to 1.6%.

The mixture of the present invention further comprises, ii) N-acetyl-cysteine (NAC), in combination with i) at least one strain of bacteria belonging to the species *Lactobacillus crispatus.*

The N-acetyl-cysteine or N-acetyl-L-cysteine or NAC (CAS No. 616-91-1) is an N-acetyl derivative of the sulphur-containing amino acid cysteine.

N-acetyl-L-cysteine is a precursor of the endogenous synthesis of glutathione and increases glutathione levels in the long term. Glutathione is one of the most important antioxidant substances in the body.

In the context of the present invention, the terms "N-acetyl-cysteine", "N-acetyl-L-cysteine" and "NAC" are used interchangeably and are intended to refer to, and include, any salt, prodrug, modification and metabolite thereof. For example, the hydrophilia of an N-acetyl-L-cysteine can be reduced by chemical modification of the carboxyl group and/or the thiol group.

For example, the N-acetyl-cysteine (NAC) is N-acetyl-L-cysteine (CAS No. 616-91-1) in the form of a 60 mesh powder.

The N-acetyl-cysteine (NAC) is present in the mixture of the invention in an amount by weight, with respect to the total weight of the mixture, preferably comprised in the range from 20% to 50%, more preferably comprised from 27% to 45%, even more preferably comprised from 31% to 33%, e.g., equal to 32.15%.

The mixture of the present invention further comprises, iii) lycopene, in combination with i) at least one strain of bacteria belonging to the species *Lactobacillus crispatus* and ii) N-acetyl-cysteine (NAC).

The lycopene, IUPAC name (6E,8E,10E,12E,14E,16E,18E,20E,22E,24E,26E)-2,6,10,14,19,23,27,31-Octamethyl-dotriaconta-2,6,8,10,12,14,16,18,20,22,24,26,30-tridecane (CAS No 502-65-8)) is an acyclic isomer hydrocarbon of beta-carotene, containing 11 conjugated and 2 unconjugated double bonds, belonging to the carotenoid group.

In nature, lycopene is found in trans isomer form, which is the most thermodynamically stable form. Given the presence of the double bonds, lycopene undergoes trans-cis isomerisation, which can be induced by light, heat and chemical reactions.

Lycopene is found both in plants and in fungi. In plants, lycopene is particularly abundant in tomatoes, and is present, in smaller amounts, also in other plants, such as watermelon, red oranges, melon, guava, pink and red grapefruit, apricots, papaya and red cabbage.

Lycopene is used as a food additive for human consumption. The EFSA (European Food Safety Authority) considers it a food colour, identified by the number E160d.

In the context of the present invention, "lycopene" is intended to refer to, and include, a lycopene in any isomeric form or isomeric mixtures, and/or a derivative thereof from any source, for example from plants, fungi and synthetic sources, also, for example, in the form of an extract containing lycopene, optionally encapsulated.

For example, the lycopene is from tomato extract (*Solarium lycopersicum L.*), in the form of microencapsulated powder with a minimum lycopene titer of 5% by weight, preferably comprised from 5% to 85%, more preferably comprised from 10% to 60%, even more preferably comprised from 15% to 35%.

Preferably, the lycopene is present in the mixture of the invention in an amount by weight, with respect to the total weight of the mixture, preferably comprised in the range from 1% to 3%, more preferably comprised from 1.5% to 2.5%, even more preferably comprised from 1.8% to 2%, e.g., equal to 1.9%.

In one embodiment, the mixture of the present invention comprises or, alternatively, consists of i) at least one strain of bacteria belonging to the species *Lactobacillus crispatus,* ii) N-acetyl-cysteine (NAC) and iii) lycopene, wherein:

- the at least one strain of bacteria belonging to the species *Lactobacillus crispatus* is present in an amount by weight, with respect to the total weight of the mixture, preferably comprised in the range from 0.5% to 2.5%, more preferably comprised from 1% to 2%, even more preferably comprised from 1.3% to 1.9%, e.g., equal to 1.6%; and/or
- the N-acetyl-cysteine (NAC) is present in an amount by weight, with respect to the total weight of the mixture, preferably comprised in the range from 20% to 50%, more preferably comprised from 27% to 45%, even more preferably comprised from 31% to 33%, e.g., equal to 32.15%; and/or
- the lycopene is present in an amount by weight, with respect to the total weight of the mixture, preferably comprised in the range from 1% to 3%, more preferably comprised from 1.5% to 2.5%, even more preferably comprised from 1.8% to 2%, e.g., equal to 1.9%.

A further object of the present invention is a mixture comprising or, alternatively, consisting of:

i) at least one strain of bacteria belonging to the species *Lactobacillus crispatus;* and
ii) N-acetyl-cysteine (NAC); and
iii) a lycopene,
and, furthermore, one or more of:
iv) vitamin D, preferably vitamin D3; and/or
v) folic acid.

Preferably, said i) at least one strain of bacteria belonging to the species *Lactobacillus crispatus* is selected from the strain of bacteria *Lactobacillus crispatus* nova LCR DSM 34348 and the strain of bacteria *Lactobacillus crispatus* GA31 DSM 35105, or mixtures thereof; preferably, each of the mentioned strains of bacteria at a concentration comprised from $1 \times 10^6$ CFU to $1 \times 10^{12}$ CFU, more preferably comprised from $1 \times 10^8$ CFU to $1 \times 10^{10}$ CFU, for example $1 \times 10^9$ CFU.

Advantageously, it has been observed that the strain of bacteria *Lactobacillus crispatus* GA31 DSM 35105 has an activity and effectiveness very similar and comparable to that of *Lactobacillus crispatus* nova LCR DSM 34348, when considered individually or in combination with the latter, when said strains of bacteria are in the presence of an NAC, a lycopene, a vitamin D and a folic acid.

In the context of the present invention, "vitamin D" is intended to refer to, and include, one or more "group D vitamins", i.e. it means one or more compounds belonging to a group of liposoluble prohormones of natural origin comprising: vitamin D1 (ergocalciferol and lumisterol 1:1), vitamin D2 (ergocalciferol), vitamin D3 (cholecalciferol); vitamin D4 (dihydroergocalciferol), vitamin D5 (sitocalciferol), and mixtures thereof.

The vitamin D is preferably vitamin D3.

Vitamin D, preferably vitamin D3, is present in the mixture of the invention, for example, in an amount by weight, with respect to the total weight of the mixture, preferably comprised from 0.002% to 2%, more preferably comprised from 0.002% to 1.5%, even more preferably comprised from 0.002% to 1%, e.g., equal to 0.006%.

Folic acid, IUPAC name (S)-2-(4-((2-amino-4-hydroxypteridin-6-yl)-methylamino)benzamide)pentanedioic acid (CAS RN 59-30-3)) is a water-soluble group B vitamin (it is in fact also known as vitamin B9). It is a compound involved in various important biochemical reactions.

In the context of the present invention, the terms "folic acid", "folate(s)" and/or "vitamin B9" are used interchangeably and are intended to include and refer to the many forms of folic acid and the compounds related thereto, including tetrahydrofolic acid (the active form), methyltetrahydrofolate (the primary form found in blood), methenyltetrahydrofolate, folinic acid, folacin and pteroylglutamic acid and/or a salt thereof and/or mixtures of salts thereof.

Folic acid is present in the mixture of the invention in an amount by weight, with respect to the total weight of the mixture, preferably comprised from 20% to 90%, more preferably comprised from 40% to 80%, even more preferably comprised from 60% to 70%, e.g., equal to 64.3%.

In one embodiment, the mixture of the present invention comprises or, alternatively, consists of i) at least one strain of bacteria belonging to the species *Lactobacillus crispatus,* ii) N-acetyl-cysteine (NAC), iii) lycopene, iv) vitamin D, preferably vitamin D3, and v) folic acid, wherein:

- the at least one strain of bacteria belonging to the species *Lactobacillus crispatus* is present in an amount by weight, with respect to the total weight of the mixture, preferably comprised in the range from 0.5% to 2.5%, more preferably comprised from 1% to 2%, even more preferably comprised from 1.3% to 1.9%, e.g., equal to 1.6%; and/or
- the N-acetyl-cysteine (NAC) is present in an amount by weight, with respect to the total weight of the mixture, preferably comprised in the range from 20% to 50%, more preferably comprised from 27% to 45%, even more preferably comprised from 31% to 33%, e.g., equal to 32.15%; and/or
- the lycopene is present in an amount by weight, with respect to the total weight of the mixture, preferably comprised in the range from 1% to 3%, more preferably comprised from 1.5% to 2.5%, even more preferably comprised from 1.8% to 2%, e.g., equal to 1.9%; and/or
- the vitamin D, preferably vitamin D3, is present in the mixture of the invention in an amount by weight, with respect to the total weight of the mixture, preferably comprised from 0.002% to 2%, more preferably comprised from 0.002% to 1.5%, even more preferably comprised from 0.002% to 1%, e.g., equal to 0.006%; and/or
- the folic acid is present in the mixture of the invention in an amount by weight, with respect to the total weight of the mixture, preferably comprised from 20% to 90%, more preferably comprised from 40% to 80%, even more preferably comprised from 60% to 70%, e.g., equal to 64.3%.

A further object of the present invention is a composition comprising the mixture according to the invention and, optionally, at least one acceptable pharmaceutical or food grade additive and/or excipient.

In one embodiment of the present invention, said composition is formulated for oral administration, preferably as a lozenge, capsule, tablet, granular powder, hard-shell capsule, mouth-dissolving granules, sachet or pill, suspensions (for example drinkable vials) or solutions (monophasic or biphasic). Alternatively, the composition is prepared extemporaneously by mixing the composition, for example, with water, milk, fruit juice or another drinkable liquid.

The compositions of the invention can be pharmaceutical compositions, compositions for medical devices, dietary supplements, foods, novel foods, probiotic products or compositions for foods for special medical purposes (FSMPs).

In the context of the present invention, the term "medical device" is used within the meaning according to Legislative Decree n° 46 of 24 February 1997, or in accordance with the new Medical Devices Regulation (EU) 2017/745 (MDR).

In the context of the present invention, the term "novel food" is used within the meaning according to Regulation (EC) 258/97.

In the context of the present invention, the expression "at least one pharmaceutical or food grade additive and/or excipient" means a substance devoid of therapeutic activity adapted for pharmaceutical use or food use. In the context of the present invention, the additives and/or excipients acceptable for pharmaceutical or food use include all the auxiliary substances known to the person skilled in the art for the preparation of compositions in solid, semisolid or liquid form, such as, for example, diluents, solvents, solubilisers, acidifiers, thickeners, sweeteners, flavour enhancers, colourants, lubricants, surfactants, preservatives, pH stabilising buffers and mixtures thereof. The total daily doses of active ingredients provided by the composition comprising the mixture of the invention are shown in Table 1 below.

Table 1

| Active ingredient | Dosage/day |
|---|---|
| *Lactobacillus crispatus:* the strain of bacteria *Lactobacillus crispatus* nova LCR DSM 34348 and/or the strain of bacteria *Lactobacillus crispatus* GA31 DSM 35105; e.g., $1\times10^9$ CFU | from 5 mg to 20 mg, e.g., 10 mg |
| | |
| N-acetyl-cysteine (NAC) | from 100 mg to 300 mg, e.g., 200 mg |
| Lycopene | from 5 mg to 20 mg, e.g., 12 mg |
| **Optional active ingredient** | **Dosage/day** |
| vitamin D, preferably vitamin D3 | from 25 μg to 50 μg, e.g., 40 μg |

(continued)

| Optional active ingredient | Dosage/day |
|---|---|
| folic acid | from 250 mg to 500 mg, e.g., 400 mg |

Unless otherwise specified, the expression composition or mixture or anything else comprising a component in an amount "comprised in a range from x to y" is used to indicate that said component can be present in the composition or mixture or extract or anything else in all the amounts present in said range, even if not specified, including the endpoints of the range.

A further object of the present invention is the use of the mixture and/or the compositions containing it of the invention in a method of treatment, preventive and/or curative, of female infertility, particularly to promote and enhance female gametogenesis, by promoting folliculogenesis, oocyte maturation, ovulation and the luteal phase, and thus promoting the development of healthy mature gametes.

A further object of the present invention is the use of the mixture and/or the compositions containing it of the invention in a method, preventive and/or curative, to promote and enhance female gametogenesis, promote folliculogenesis, oocyte maturation, ovulation and the luteal phase, stimulate cell viability and the antioxidant mechanisms of ovarian cells, reduce the inflammatory state and apoptotic mechanisms of ovarian cells, promote the transition of meiosis from metaphase I to anaphase I in oocytes, maintain proper function of hormones responsible for gamete maturation, and promote the development of healthy mature gametes, in subjects suffering from female infertility.

The invention is further described by the examples that follow, to be understood as given purely by way of illustration and in no way limiting the scope of protection of the invention.

**EXAMPLES**

Two strains of bacteria belonging to the species *Lactobacillus crispatus* were tested: the strain *Lactobacillus crispatus* nova LCR deposited by Probionova SA on 26.07.2022 at the *Deutsche Sammlung von Mikroorganismen und Zellkulturen* (DSMZ) under deposit number DSM 34348, at a concentration of $1 \times 10^9$ CFU, and the strain *Lactobacillus crispatus GA31* deposited by Probionova SA on 17.07.2024 at the *Deutsche Sammlung von Mikroorganismen und Zellkulturen* (DSMZ) under deposit number DSM 35105, at a concentration of $1 \times 10^9$ CFU.

The following mixtures were prepared.

Example 1

Table 2

| Active ingredient | Amount total 222 mg |
|---|---|
| *Lactobacillus crispatus:* the strain of bacteria *Lactobacillus crispatus* nova LCR DSM 34348 and/or the strain of bacteria *Lactobacillus crispatus* GA31 DSM 35105; e.g., $1 \times 10^9$ CFU | 10 mg, or 15 mg |
| NAC | 200 mg |
| tomato extract with a lycopene titerof 5% by weight | 12 mg or 20 mg |

Example 2

Table 3

| Active ingredient | Amount tot. 622.04 mg |
|---|---|
| *Lactobacillus crispatus:* the strain of bacteria *Lactobacillus crispatus* nova LCR DSM 34348 and/or the strain of bacteria *Lactobacillus crispatus* GA31 DSM 35105; e.g., $1 \times 10^9$ CFU | 10 mg, or 15 mg |
| NAC | 200 mg |

(continued)

| Active ingredient | Amount tot. 622.04 mg |
|---|---|
| tomato extract with a lycopene titer of 5% by weight | 12 mg or 20 mg |
| vitamin D3 | 40 μg |
| folic acid | 400 mg |

The first experimental results preliminarily indicate that the mixtures and compositions containing it (mixtures and compositions of the invention) could have useful application for treating, in a preventive and/or curative manner, female infertility, particularly to promote and enhance female gametogenesis, by promoting folliculogenesis, oocyte maturation, ovulation and the luteal phase, and thus promoting the development of healthy mature gametes.

In particular, the mixtures and compositions containing it (mixtures and compositions of the invention) are effective in promoting and enhancing female gametogenesis, promoting folliculogenesis, oocyte maturation, ovulation and the luteal phase, stimulating cell viability and the antioxidant mechanisms of ovarian cells, reducing the inflammatory state and apoptotic mechanisms of ovarian cells, promoting the transition of meiosis from metaphase I to anaphase I in oocytes, maintaining proper function of the hormones responsible for gamete maturation, and promoting the development of healthy mature gametes, in subjects suffering from female infertility.

## Experimental section

Two strains of bacteria belonging to the species *Lactobacillus crispatus* were tested: the strain *Lactobacillus crispatus* nova LCR deposited by Probionova SA on 26.07.2022 at the *Deutsche Sammlung von Mikroorganismen und Zellkulturen* (DSMZ) under deposit number DSM 34348, at a concentration of $1 \times 10^9$ CFU, and the strain *Lactobacillus crispatus GA31* deposited by Probionova SA on 17.07.2024 at the *Deutsche Sammlung von Mikroorganismen und Zellkulturen* (DSMZ) under deposit number DSM 35105, at a concentration of $1 \times 10^9$ CFU. The development of innovative treatment options for restoring proper maturation of female gametes required both the study of the passage of substances accross the gastrointestinal barrier and a thorough understanding of the cellular and molecular mechanisms involved at the level of the gut-ovary axis. For this purpose, a two-step study was conducted: a first step **(Step 1)** aimed at analysing the absorption mechanisms and bioavailability underlying the passage of substances through the intestinal tract, by exploiting a 3D barrier model validated in the literature; and a second step **(Step 2)** aimed at evaluating the biological activity and the beneficial effect of the novel mixtures/compositions, after intestinal absorption, directly in an *in vitro* model of female infertility.

## 1. MATERIALS AND METHODS

### 1. 1 *Cell cultures*

The human intestinal epithelial cell line CaCo-2, purchased from the American Type Culture Collection (ATCC), was used as an experimental model to predict the characteristics of intestinal absorption following oral administration. This cell line was cultured in Dulbecco's Modified Eagle Medium/Nutrient F-12 Ham (DMEM-F12, Merck Life Science, Rome, Italy) containing 10% FBS (Merck Life Science, Rome, Italy), 2 mM of L-glutamine and 1% penicillin-streptomycin (Merck Life Science, Rome, Italy) and kept in an incubator at 37 °C and 5% CO2. The experiments were conducted using cells with a passage number comprised between 26 and 32 to maintain the correct properties of permeability and paracellular transport. The cells were seeded in various ways, including $1 \times 10^4$ cells in 96-well plates, in order to study cell viability and ROS production, synchronising the cells for 8 hours with DMEM without phenol red and supplemented with 0.5% FBS, 2 mM L-glutamine and 1% penicillin-streptomycin at 37 °C; $2 \times 10^4$ cells in Transwell® 6.5 mm plates (Corning® Costar®, Merck Life Science, Rome, Italy) with a polycarbonate membrane insert with 0.4 μm pores (Corning® Costar®, Merck Life Science, Rome, Italy) in a 24-well plate in order to perform the absorption analyses.

The human ovarian surface epithelial cell line HOSEpiC, purchased from ScienCell (Carlsbad, California, United States), was cultured in Ovarian Epithelial Cell Medium (Carlsbad, California, United States) supplemented with 1% of a specific supplement for the growth of the ovarian epithelial cells (Carlsbad, California, United States) and 1% penicillin-streptomycin (Carlsbad, California, United States) and incubated at 37 °C, 5% CO2 and 95% humidity. When the cells reached 80% of confluence, they were plated as follows: $1 \times 10^4$ cells in a 96-well multiwell in order to study cell viability and ROS; $5 \times 10^5$ cells in a polylysine-coated 6-well multiwell in order to study the various markers with an ELISA kit; $1 \times 10^5$ cells in a polylysine-coated 24-well multiwell, in which the Transwell® supports containing the CaCo-2 cells were placed.

*1.2 Preparation of the substances*

*Lactobacillus Crispatus* (L. *Crispatus*) donated by Probionova (Lugano, Switzerland), Ribomix (Prosol Spa, Madone BG), folic acid, NAC, lycopene and vitamin D (Prosol Spa, Madone BG) were prepared at the time of the stimulations. *L. Crispatus* was reconstituted in Dulbecco's Modified Eagle Medium without phenol red (DMEM, Merck Life Science, Rome, Italy), supplemented with 0% FBS, 50 UI/ml of penicillin-streptomycin (Merck Life Science, Rome, Italy) and L-glutamine 2mM solution (Merck Life Science, Rome, Italy). For every test, the samples were diluted in culture medium prior to use, in order to reach a final concentration of $1*10^9$ colony-forming units (CFU)/ml of probiotics, which correspond to 10mg/ml. Folic acid, NAC, lycopene and vitamin D were similarly prepared in DMEM without phenol red (Merck Life Science, Rome, Italy), supplemented with 0% FBS, 50 UI/ml of penicillin-streptomycin (Merck Life Science, Rome, Italy) and L-glutamine 2mM solution (Merck Life Science, Rome, Italy).

*1.3 Experimental protocol*

With the aim of developing innovative treatment options for restoring proper maturation of the oocytes, the project was divided into two steps: a first preliminary step aimed at analysing the absorption mechanisms and bioavailability following the passage of new substances through the intestinal tract by exploiting a 3D barrier model validated in the literature, and a second step aimed at studying the effect of the novel combinations, after intestinal absorption, directly in a model of female infertility. Firstly, permeability was analysed by measuring the transepithelial/transendothelial electrical resistance (TEER), a widely accepted quantitative technique that exploits a volt/ohm meter (EVOM3) to measure the integrity of the dynamics of tight junctions, by evaluating the integrity of the cellular barriers following the passage of the novel formulation. Furthermore, the changes in the expression of tight junction (TJ) proteins were evaluated in order to analyse the barrier function, since, behaving like multiple protein complexes, they form a selectively permeable seal between adjacent epithelial cells and, for this reason, they are analysed as indicators of the integrity of the epithelium, in particular by analysing occludin, claudin and zonula occludens. Subsequently, a co-culture was prepared, made up of CaCo-2 intestinal cells previously used in step 1, positioned in the apical compartment of Transwell® inserts, and HOSEpiC ovary cells, seeded in the basolateral compartment, to create a gut-ovary axis capable of perfectly mimicking what happens in a human being. Moreover, once the ovarian monolayer was obtained, the conditions of oxidative stress were mimicked *in vitro.* With this model, the effect of a novel therapeutic approach was studied by first analysing cell viability and mitochondrial metabolism to verify the absence of oxidative stress and ROS production. This is because ovarian cells and oocytes are susceptible to oxidative stress. In this context, an evaluation was also made of the apoptotic process, in particular of BAX, caspase 3 and cytochrome C. Furthermore, an analysis was performed of the inflammatory state by evaluating COX2 and prostaglandin activity. An analysis was also performed of GDF9 and BMP15, factors secreted by the oocyte with a leading role in controlling ovarian function in female reproduction, and oestradiol and progesterone, which act locally to modulate ovarian function.

*1.4 MTT assay*

The analysis of cell viability was carried out with a classic technique based on the MTT in vitro toxicology assay kit (Merck Life Science, Rome, Italy), following the manufacturer's instructions. In fact, at the end of the treatment, the cells were incubated with 1% MTT dye for 2 hours in an incubator at 37 °C, 5% CO2 and 95% humidity; the purple formazan crystals were then dissolved in an equal volume of MTT solubilisation solution. Absorbance was analysed with a spectrophotometer (Infinite 200 Pro MPlex, Tecan, Männedorf, Switzerland) at 570 nm with correction at 690 nm, and the results were expressed versus the control (0% line), which represented the untreated cells. The results represent the percentage of viable cells versus the control, which makes it possible to understand whether the stimulation is safe or not.

*1.5 ROS production*

A quantification of the release of superoxide anions was obtained following a standard protocol based on reduction of cytochrome C, and absorbance in the culture supernatants was measured at 550 nm using a spectrophotometer (Infinite 200 Pro MPlex, Tecan, Männedorf, Switzerland). In particular, 100 µL of cytochrome C (Merck, Milan, Italy) were added to all the wells, whilst 100 µL of superoxide dismutase (Merck, Milan, Italy) and 100 µL of cytochrome C were added to the empty wells; the plate was then incubated for 30 minutes. Subsequently, 100 µL were drawn from each well and the absorbance was measured with a spectrophotometer (Infinite 200 Pro MPlex, Tecan, Männedorf, Switzerland) at 550 nm. The rate of O2 was expressed as mean $\pm$ SD (%) of nanomoles of reduced cytochrome C per microgram of protein versus the control (0 line).

*1.6 In vitro model of intestinal barrier*

For the purpose of studying the substances under examination, an in vitro model of an intestinal barrier was created using the Transwell® system, following a standard protocol reported in the literature and approved by the European Medicines Agency (EMA) and by the Food and Drug Administration (FDA) to predict the absorption, metabolism and bioavailability of different substances after oral administration in human beings. Briefly, the CaCo-2 cells, plated as described previously, were maintained in a complete culture medium, which was changed every other day on the basal side and on the apical side for 21 days before stimulation. During the whole maturation period, the TEER values were evaluated by EVOM3, coupled with STX2 electrodes with sticks (World Precision Instruments, Sarasota, FL, USA), in order to evaluate the formation of mature intestinal epithelia and a correct paracellular mechanism. On the 21st day, when the TEER values were $\geq 400\,\Omega cm2$ [12], the analysis of absorption began. Before stimulation, on the apical side, the culture medium was brought to pH 6.5, the pH of the lumen of the small intestine, whereas the pH of 7.4 on the basolateral side represented blood. Under these conditions, the substances under examination were added to the apical environment in a time-dependent study (from 2 to 6h) and, at every time point, the intestinal absorption or bioavailability was measured by means of 0.04% fluorescein (sigma), a marker dye applied to evaluate transepithelial transport. The amount of fluorescein transported was measured at 37 °C for 40 minutes by incubating CaCo-2 cells at the above-mentioned concentration (apical pH, 6.0; basolateral pH, 7.4). The fluorescence was measured with a spectrophotometer (Infinite 200 Pro MPlex, Tecan, Männedorf, Switzerland) at the excitation/emission wavelengths of 490/514 nm.

The cells were stimulated with all the substances from 2 hours to 6 hours before the subsequent analyses, including the permeability assay. The permeation velocity was determined with the following formula:

$$Jmax\ [C]/(Kt + [C])$$

wherein:

    C: initial probe concentration
    Jmax: passage of fluorescence.
    Kt: Michaelis-Menten constant

The data obtained were converted into absorption, using the formula for the permeability index:

$$Papp = dQ/dt \twoheadrightarrow 1/m0 \twoheadrightarrow 1/A \twoheadrightarrow V\ Donor$$

wherein:

    dQ: amount of substance transported (nmol or $\mu$g);
    dt: incubation time (s);
    m0: amount of substrate applied to the donor compartment (nmol or $\mu$g);
    A: surface area of the Transwell® membrane ($cm^2$);
    VDonor: volume of the donor compartment ($cm^3$).

Negative controls without cells were analysed to rule out the influence of the Transwell® membrane. The analysis was performed in triplicate and reproduced five times.

*1.7 Claudin-1 ELISA kit*

The activity of human claudin-1 was measured in the CaCo-2 lysates by means of an ELISA kit (Cusabio Technology LLC, Huston, Houston, TX, USA), following the manufacturer's instructions. The CaCo-2 cells were lysed with cold PBS (Merck Life Science, Rome, Italy) $1\times$, centrifuged at $1500\times$ g for 10 minutes at 4 °C and 100 $\mu$L of each sample were analysed and read at 450 nm by means of a spectrometer (Infinite 200 Pro MPlex, Tecan, Männedorf, Switzerland). The results were obtained by comparing the data with the standard curve (which goes from 0 to 1000 pg/mL) and were expressed as mean $\pm$ SD (%) versus the control (0 line) of five independent experiments carried out in triplicate.

*1.8 Occludin quantification assay*

The human occludin ELISA kit (OCLN) (MyBiosource, San Diego, CA, USA) was used according to the manufacturer's instructions. The CaCo-2 cells were lysed with cold phosphate-buffered saline (PBS, Merck Life Science, Rome, Italy) 1×, centrifuged at 1500× g for 10 minutes at 4 °C and 100 μL of each sample were analysed. The enzymatic reaction was measured by means of a spectrometer (Infinite 200 Pro MPlex, Tecan, Männedorf, Switzerland) at 450 nm. The results were obtained by comparing the data with the standard curve (which goes from 0 to 1500 pg/mL) and were expressed as a percentage (%) versus the control (0 line) of five independent experiments carried out in triplicate.

*1.9 Analysis of human tight junction protein 1 (ZO-1)*

The human tight junction protein 1 (TJP1) ELISA kit (MyBiosource, San Diego, CA, USA) was used following the manufacturer's instructions. The CaCo-2 cells were lysed with cold PBS (Merck Life Science, Rome, Italy) 1×, centrifuged at 5000× g for 5 minutes at 4 °C and 100 μL of each sample were analysed. The plates were read by means of a spectrometer (Infinite 200 Pro MPlex, Tecan, Männedorf, Switzerland) at 450 nm. The data were obtained and compared with the standard curve (from 0 to 1000 pg/mL) and the results were expressed as mean ± SD (%) versus the control (0 line) of five independent experiments carried out in triplicate.

*1.10 SOD activity*

The SOD level was measured following the manufacturer's instructions (Cayman's Superoxide Dismutase Assay Kit; Tallinn, Estonia); the kit detects all three types of SOD (Cu/Zn, Mn and FeSOD). Briefly, 200 μL of radical detector and 10 μL of suitably diluted standard were added into the standard well; 200 μL of radical detector and 10 μL of the tested sample were added into the sample wells. After the addition of 20 μL of xanthine oxidase in all the wells, the plate was gently shaken and incubated for 30 minutes. Finally, the level of SOD present in the 3D cell lysates was measured by comparing the data with a standard curve (0.05-0.005 U/mL). The absorbance of all the samples was measured with a spectrometer (Infinite 200 Pro MPlex, Tecan, Männedorf, Switzerland) at 440-460 nm and the results were expressed as mean (%) versus the control.

1.11 *Measurement of NO production*

The production of nitric oxide (NO) was evaluated by indirectly measuring the concentration of nitrites using the Griess assay (Promega Corporation, Madison, WI, USA) following the manufacturer's instructions, as previously described. The absorbance was measured by a microplate reader (Infinite 200 Pro MPlex, Tecan, Männedorf, Switzerland) at 550 nm. For calibration of the assay, standards of sodium nitrite were used in the range of 100-3125 μM (serial dilution two times).

*1.12 Assay for BAX activity*

The activity of BAX was determined using an ELISA kit (Human Bax ELISA Kit, MyBiosource, San Diego, CA, USA) according to the manufacturer's instructions. The absorbance of the samples was measured at 450 nm with a spectrophotometer (Infinite 200 Pro MPlex, Tecan, Männedorf, Switzerland) and the results were compared with the standard curve (range from 0 to 2000 pg/mL) and expressed as mean ± SD (%) normalised to the control value (0 line).

*1.13 Caspase 3 assay*

The activity of caspase 3 was studied in the lysates by means of an ELISA kit (Caspase 3 (Cleaved) Human ELISA Kit, Thermoscientific, Waltham, MA, USA), according to the manufacturer's instructions, reading the absorbance of the sample at 450 nm with a spectrometer (Infinite 200 Pro MPlex, Tecan, Männedorf, Switzerland). The data were obtained by comparison with a standard curve (from 0.039 to 2.5 ng/mL) and the results were expressed as mean ± SD (%) versus the control value (0 line).

*1.14 Cytochrome C ELISA assay*

The activity of Cytochrome C was studied in the lysates by means of an ELISA kit (Cytochrome C Human ELISA Kit, Thermoscientific, Waltham, MA, USA), according to the manufacturer's instructions, reading the absorbance of the sample at 450 nm with a spectrometer (Infinite 200 Pro MPlex, Tecan, Männedorf, Switzerland). The data were obtained by comparison with a standard curve (from 0.078 to 5 ng/mL) and the results were expressed as mean ± SD (%) versus the control value (0 line).

*1.15 COX2 ELISA assay*

The Human COX2 ELISA Kit was used for the quantitative determination of COX2, following the manufacturer's instructions (Abcam, Cambridge, United Kingdom). The concentration was calculated by comparing the results with the standard curve (generated by the positive control) (from 1.229 to 300 ng/mL according to different serial dilutions) and shown as mean $\pm$ SD (%) versus the control (0 line).

*1.16 Prostaglandin E2 ELISA assay*

The activity of Prostaglandin E2 was studied in the supernatant by means of an ELISA kit (Prostaglandin E2 Human ELISA Kit, Thermoscientific, Waltham, MA, USA), according to the manufacturer's instructions, reading the absorbance of the sample at 450 nm with a spectrometer (Infinite 200 Pro MPlex, Tecan, Männedorf, Switzerland). The data were obtained by comparison with a standard curve (da 31.3 a 4000 pg/mL) and the results were expressed as mean $\pm$ SD (%) versus the control value (0 line).

*1.17 CDK1 ELISA assay*

The CDK1 ELISA kit (MyBiosource, San Diego, CA, USA) was used according to the manufacturer's instructions. The HOSEpiC cells were lysed with cold phosphate-buffered saline (PBS, Merck Life Science, Rome, Italy) 1×, centrifuged at 5000× g for 5 minutes and 100 µL of each sample were analysed by reading the absorbance of the sample at 450 nm with a spectrometer (Infinite 200 Pro MPlex, Tecan, Männedorf, Switzerland). The results were obtained by comparing the data with the standard curve (ranging from 0.156 to 10 ng/mL) and were expressed as a percentage (%) versus the control (0 line) of five independent experiments carried out in triplicate.

*1.18 GDF9 ELISA assay*

The GDF9 ELISA kit (MyBiosource, San Diego, CA, USA) was used according to the manufacturer's instructions. The HOSEpiC cells were lysed with cold phosphate-buffered saline (PBS, Merck Life Science, Rome, Italy) 1×, centrifuged at 1500× g for 10 minutes at 4°C and, finally, 100 µL of each sample were analysed by reading the absorbance of the samples at 450 nm with a spectrometer (Infinite 200 Pro MPlex, Tecan, Männedorf, Switzerland). The results were obtained by comparing the data with the standard curve (which goes from 0.16 to 10 ng/mL) and were expressed as a percentage (%) versus the control (0 line) of five independent experiments carried out in triplicate.

*1.19 BMP-15 ELISA assay*

The BMP-15 ELISA kit (Thermoscientific, Waltham, MA, USA) was used according to the manufacturer's instructions. In detail, 100 µL of standard or of sample were added into the wells and allowed to incubate for 2.5 h. After the wells had been washed 4 times, 100 µL of biotin conjugate were added and the plate was incubated for one hour. After 3 further washes, 100 µL of Streptavidin-HRP solution were added for 45 minutes. Another 3 washes followed and 100µL of TMB Substrate were added. After 30 minutes, 50 µL of stop solution were added and the plate was read at 450 nm with a spectrometer (Infinite 200 Pro MPlex, Tecan, Männedorf, Switzerland). The results were obtained by comparing the data with the standard curve (which goes from 0.6 to 150 ng/mL) and were expressed as a percentage (%) versus the control (0 line) of five independent experiments carried out in triplicate.

*1.20 Progesterone ELISA assay*

The determination of progesterone was studied in the supernatant by means of an ELISA kit (Progesterone Competitive ELISA Kit, Thermoscientific, Waltham, MA, USA), according to the manufacturer's instructions, reading the absorbance of the sample at 450 nm with a spectrometer (Infinite 200 Pro MPlex, Tecan, Männedorf, Switzerland). The data were obtained by comparison with a standard curve (da 50 a 3200 pg/mL) and the results were expressed as mean $\pm$ SD (%) versus the control value (0 line).

*1.21 Oestradiol ELISA assay*

The determination of progesterone was studied in the cell lysate by means of an ELISA kit (Estradiol ELISA Kit (Competitive EIA) - LS-F5297, LSBio, Seattle, DC, USA) following the manufacturer's instructions. Briefly, after the stimulations the cells were collected, lysed by freezing and thawing 3 times and centrifuged at 1500×g for 10 minutes at 2-8 °C. A volume of 50 µl of each sample was incubated with 50 µl detection reagent A for 60 min at 37 °C and then with 100

μl of detection reagent B for 30 min at 37 °C. Subsequently, 90μl of TMB Substrate were added for 15 minutes at 37 °C in darkness and then 50μl of stop solution were added into every well. The absorbance was measured at 450 nm with a plate reader (Infinite 200 Pro MPlex, Tecan, Cernusco sul Naviglio, MI, Italy) and the results were generated on the basis of a standard curve (from 12.35 to 1000 pg/mL) and expressed as ng/pl.

*1.22 Statistical Analyses*

The collected data were processed with Prism GraphPad 9.4.1 statistical software (GraphPad Software, La Jolla, CA, USA) using one-way analysis of variance (ANOVA), followed by the Bonferroni post hoc test. The comparisons between the two groups were carried out using a two-tailed Student t test. The multiple comparisons between groups were analysed with a two-way ANOVA followed by Dunnett's two-sided post hoc test. All the results were expressed as mean $\pm$ SD of at least 5 independent experiments carried out in triplicate. The differences with $p < 0.05$ were considered statistically significant.

## 2. RESULTS

*2.1 STEP 1 - Dose-response evaluation on intestinal cells and evaluation of function at the level of the intestinal barrier*

With the aim of evaluating what the best concentration of each selected substance might be, and to prevent any cytotoxic effect, the viability of the CaCo-2 cells was analysed by means of the MTT assay.

<u>Tested concentrations:</u>

- *L. crispatus* (10 mg/ml; 20 mg/ml; 30 mg/ml; 40 mg/ml; 50 mg/ml)
- NAC (0.3 mM; 0.6 mM; 1 mM)
- lycopene (0.1 μM; 0.2 μM; 0.5 μM)
- vitamin D3 (10 nM; 100 nM)
- folic acid (10 μM; 50 μM; 125 μM; 500 μM)

As shown in **Figure 1,** all the tested concentrations showed an increase in cell viability (mitochondrial metabolism) versus the control (p<0.05). In particular, 10 mg/ml of *L. crispatus,* NAC 0.6 mM, lycopene 0.2 μM, vitamin D3 10 nM and folic acid 50 μM are the most effective concentrations and statistically significative (p<0.05) compared to the other tested doses. Therefore, these were maintained for all the subsequent experiments.
In order to test the single substances and mixtures of the invention through the intestinal barrier, a 3D *in vitro* model (authorised by EMA/FDA) of the intestinal barrier was used. The following were evaluated:

- safety and toxicity;
- oxidative stress;
- barrier integrity;
- prediction of absorption and permeability;
- activity of the tight junctions.

Accordingly, various experiments were carried out on the CaCo-2 cell line to rule out any toxic effect or irritability and confirm the absence of oxidative stress.
The following mixture of the invention was tested:

- MIX <u>(Example 2):</u> 10 mg/ml of *L. crispatus,* NAC 0.6 mM, lycopene 0.2 μM, vitamin D3 10 nM and folic acid 50 μM.

Based on the results obtained, it can be assumed that all the substances and the selected mixture are capable of positively influencing the intestinal barrier without side effects.
As shown in **Figure 2,** the results on cell viability confirmed the dosages selected from the initial screening, as they succeeded in maintaining the homeostasis of intestinal cells. MIX was confirmed to have a better effect throughout the whole period analysed compared both to the single substances and to the untreated control ($p < 0.05$), suggesting that the substances under examination, if combined, amplify their biological effect by means of a synergistic mechanism of action.
Furthermore, the antioxidant property of the mixture was confirmed, which amplified the beneficial effect compared both to the control and to the single substances (p < 0.05).

The tested mixture (MIX) is thus also capable of improving oxidative stress by reducing the production of ROS compared both to the single substances and to the control. Therefore, it is possible to affirm that the tested mixture (MIX) maintains cellular homeostasis without toxic effects, in the absence of irritability of the epithelium and without inducing oxidative stress, suggesting a synergistic effect exerted by the sum of the single substances.

In order to evaluate and analyse the proper function of the intestinal barrier, tests were conducted to mimic the complexity of the human intestinal barrier by measuring the TEER values and TJ activity. The transepithelial electrical resistance (TEER) is a classic indicator of the strength of the TJs and reflects the ionic conductance of the paracellular pathway. TEER values $\geq 400\Omega/cm^2$ indicate proper function of the TJs and of the intestinal transport mechanisms. As shown in **Figure 3,** the transepithelial electrical resistance (TEER) values of the CaCo-2 intestinal cells are higher than the accepted threshold value for a correct activity of the intestinal barrier ($>400\Omega/cm^2$). The data obtained show that the intestinal metabolism has a physiological pattern. In particular, the analysis of the intestinal epithelium demonstrates that all the single substances and mixtures tested were capable of maintaining epithelial integrity versus the control (p < 0.05), while increasing the ion flow of paracellular exchanges through the intestinal barrier. The analysis supports the observed absorption, also in terms of the kinetics of permeation of the intestinal wall. It may be observed that the peak at 4h begins to fall at 4h, whilst the increase starts to be seen at 2h. Once again, the best effect was obtained following stimulation with the tested mixture (MIX) (p < 0.05) during all the stimulation times, with a maximum peak at 4h.

The analysis of the protein-mediated TJs also confirmed the results obtained with TEER. In particular, an analysis was made of ZO-1, which maintains and simultaneously modulates the integrity of the barrier, claudin, the main protein forming the barrier, and occludin, which contributes to the stabilisation and optimal function of the barrier. The results shown in Figure 4 demonstrated that, with a better effect compared to the single substances (p<0.05), the tested mixture (MIX), has a positive influence on the TJs. This test allowed for determining the barrier integrity and function (avoiding irritability or conditions that may lead to leaky gut syndrome), confirming the active role of the TJs in the absorption of compounds. The analysis was conducted at 6h after the end the stimulation to verify that there were no alterations in the intestinal structures.

Based on these results, it can be assumed that the substances and the selected mixture are capable of improving the rate of absorption, with a more physiological behaviour of the mixture compared to the single substances (p<0.05), maintaining epithelial integrity and increasing the ion flow of paracellular exchanges through the intestinal epithelium. Furthermore, the data obtained from the analysis of the TJs demonstrated that the tested mixture (MIX) maintains the proper functions of the intestinal epithelium with a better effect compared to the single substances (p<0.05).

In order to obtain further information on intestinal absorption, further investigations were carried out by analysing the permeability and absorption values. The data of the permeability analysis (fluorescent probe) are shown in Table 4 below:

|  | 1h | 2h | 3h | 4h | 5h | 6h |
|---|---|---|---|---|---|---|
| Folic acid 50µM | $0.21\times10^{-6}$ | $0.27\times10^{-6}$ | $0.30\times10^{-6}$ | $0.34\times10^{-6}$ | $0.32\times10^{-6}$ | $0.26\times10^{-6}$ |
| NAC 0.6mM | $0.20\times10^{-6}$ | $0.25\times10^{-6}$ | $0.27\times10^{-6}$ | $0.31\times10^{-6}$ | $0.28\times10^{-6}$ | $0.2\times10^{-6}$ |
| Lycopene 0.2µM | $0.22\times10^{-6}$ | $0.28\times10^{-6}$ | $0.37\times10^{-6}$ | $0.44\times10^{-6}$ | $0.38\times10^{-6}$ | $0.32\times10^{-6}$ |
| Vitamin D 10ng | $0.33\times10^{-6}$ | $0.39\times10^{-6}$ | $0.49\times10^{-6}$ | $0.62\times10^{-6}$ | $0.57\times10^{-6}$ | $0.52\times10^{-6}$ |
| *L. Crispatus* 10mg | $0.218\times10^{-6}$ | $0.21\times10^{-6}$ | $0.24\times10^{-6}$ | $0.26\times10^{-6}$ | $0.25\times10^{-6}$ | $0.22\times10^{-6}$ |
| MIX | $0.33\times10^{-6}$ | $0.39\times10^{-6}$ | $0.49\times10^{-6}$ | $0.62\times10^{-6}$ | $0.57\times10^{-6}$ | $0.52\times10^{-6}$ |

Table 4: Analysis of Permeability

The values shown derive from the application of the formula for calculating permeability: **Jmax [C]/(Kt + [C]),** wherein: C: initial concentration of the probe; Jmax: passage of fluorescence; Kt: Michaelis-Menten constant.

The data obtained were converted into absorption, as described above. The absorption data shown in **Figure 5** were obtained as absorption normalised on the portion of fluorescein that permeabilised the sample, deriving from the calculation of permeability. An untreated sample was used as the control in the presence of the fluorescent probe.

The data confirm a correct intestinal absorption which follows what normally occurs in a human being. In particular, the absorption of the tested mixture (MIX) shows to be greater compared to the single substances.

*2.2 STEP 2 - Evaluation of function at the level of the gut-ovary axis*

The previously described *in vitro* model of the gut-ovary axis was used to analyse:

- cell viability and mitochondrial metabolism;
- absence of oxidative stress and of ROS production and endogenous antioxidant mechanisms such as SOD;
- evaluation of the inflammatory state, in particular the production of COX2 and of prostaglandins
- evaluation of the apoptotic process
- analysis of the main markers involved in the oocyte maturation process, such as CDK1, GDF-9 and BMP-15;
- analysis of the levels of progesterone and oestradiol.

The single substances and MIX were tested (Example 2): 10 mg/ml of *L. crispatus,* NAC 0.6 mM, lycopene 0.2 $\mu$M, vitamin D3 10 nM and folic acid 50 $\mu$M.

Experiments were conducted to analyse the effects on mitochondrial metabolism, on ROS production and on endogenous antioxidant mechanisms such as the activity of SOD.

**Figure 6** shows the results obtained. The results for cell viability confirmed that all the treatments were capable of maintaining the homeostasis of the ovarian cells after intestinal passage. MIX was confirmed to have a better effect compared to the single substances ($p < 0.05$). Furthermore, there was confirmation of the antioxidant property of MIX, which was capable of balancing the endogenous antioxidant mechanisms, and which amplified the beneficial effect ($p < 0.05$) compared to the single substances.

These results suggest that MIX has a regulatory effect against reactive oxygen species, acting directly or indirectly on the ovary.

Given that the initial phases of apoptosis may involve only a slight ROS/NO imbalance in favour of the latter, the two parameters were analysed by way of comparison to rule out an activation of the death pathway. As may be observed from the data in Figure 7, the trend is balanced between the two markers, suggesting that there is no apoptotic activation.

Such data were furthered by the analysis of specific markers. BAX, caspase 3 and cytochrome C are important markers of apoptosis at the ovarian level, since an increase thereof could delay cell maturation, leading them to death, i.e. a reduction thereof could ensure a prolonging of ovarian activity. The results relating to the activity of these three pro-apoptotic molecular mechanisms are shown in Figure 8 and confirmed that all the treatments were capable of slowing cell death at the level of ovarian cells. In particular, MIX was confirmed to have a better effect compared to the single substances ($p < 0.05$).

In order to evaluate the state of wellbeing of the ovarian cells, the inflammatory profile of those cells was also investigated; for this reason, the activity of COX-2 and PGE 2 were investigated. Prostaglandin E2 (PGE 2) is an endogenous lipid mediator of inflammation and its production is regulated by the enzyme cyclooxygenase-2 (COX-2). The results relating to the activity of these two mediators of inflammation are shown in Figure 9 and have confirmed that all the treatments were capable of reducing the inflammatory state at the level of the ovarian cells. In particular, MIX was confirmed to have a better effect compared to the single substances ($p < 0.05$).

In order evaluate the effects of the single substances and of the mixture of the invention on the female gamete maturation process, the activity of the main markers involved in the oocyte maturation process (CDK1, GDF-9 and BMP-15) was investigated. Cyclin-dependent kinase CDK1 has the task of promoting the transition of meiosis from metaphase I to anaphase I in oocytes; GDF9 and BMP-15 are factors that exercise a role of controlling ovarian function in female reproduction. The results relating to the activity of these three molecular mechanisms are shown in Figure 10 and confirmed that all the treatments were capable of increasing the activity of CDK1, GDF9 and BMP-15 at the level of the ovarian cells. In particular, MIX was confirmed to have a better effect compared to the single substances ($p < 0.05$).

Finally, the levels of progesterone and oestradiol production were investigated, as they play a key role in the female gamete maturation process. Progesterone is a steroid produced by the corpus luteum, which forms at the site of the follicle after ovulation. This is essential for incorporating and nourishing the fertilised embryo in the endometrium and is thus fundamental for the success of a pregnancy. In contrast, oestradiol can induce apoptosis of ovarian cells when it interacts with phosphodiesterase 3, leading to a loss of ovarian activity, and thus a failed maturation of the follicle. The results relating to the progesterone and oestradiol production are shown in Figure 11 and confirmed that all the treatments were capable of increasing the production thereof at the level of the ovarian cells.

In particular, MIX was confirmed to have a better effect compared to the single substances ($p < 0.05$).

**Conclusions**

In conclusion, based on the data obtained it is possible to highlight that the novel mixture of the invention, by virtue of the synergistic effect which amplifies the effect of the single substances, is capable of:

- maintaining the safety of the intestinal epithelium;
- maintaining unaltered the permeability related to the opening/closing of the tight junctions;
- increasing the ion flow of the intestinal barrier to facilitate absorption;
- stimulating cell viability and antioxidant mechanisms, thereby guaranteeing protection against oxidative stress and proper mitochondrial metabolism of ovarian cells;
- reducing the inflammatory state and apoptotic mechanisms that lead to the death of ovarian cells and the failed maturation of female gametes;
- promoting the transition of meiosis from metaphase I to anaphase I in oocytes, exercising a role of controlling ovarian function;
- maintaining proper function of the hormones responsible for gamete maturation;

demonstrating safety and biological effectiveness.

Finally, it was seen that both the strain of bacteria *Lactobacillus crispatus* nova LCR deposited at the *Deutsche Sammlung von Mikroorganismen und Zellkulturen* (DSMZ) under deposit number DSM 34348 and the strain of bacteria *Lactobacillus crispatus* GA31 deposited at the *Deutsche Sammlung von Mikroorganismen und Zellkulfuren* (DSMZ) under deposit number DSM 35105, when each is taken individually or in combination with the other, show a behaviour that is very similar and comparable in terms of activity and effectiveness, when said strains of bacteria are present together with an NAC, a lycopene, a vitamin D and a folic acid (MIX, Example 2).

**Claims**

1. A mixture comprising or, alternatively, consisting of:

   (i) at least one strain of bacteria belonging to the species *Lactobacillus crispatus,* preferably *Lactobacillus crispatus* nova LCR deposited at the *Deutsche Sammlung von Mikroorganismen und Zellkulturen* (DSMZ) under deposit number DSM 34348 and/or *Lactobacillus crispatus* GA31 deposited at the *Deutsche Sammlung von Mikroorganismen und Zellkulturen* (DSMZ) under deposit number DSM 35105; and
   (ii) N-acetyl-cysteine (NAC) and/or salts thereof; and
   (iii) a lycopene; and
   (iv) vitamin D, preferably vitamin D2 and/or D3; and/or
   (v) folic acid.

2. The mixture according to claim 1, wherein said at least one strain of bacteria belonging to the species *Lactobacillus crispatus* is the strain *Lactobacillus crispatus* nova LCR deposited at the *Deutsche Sammlung von Mikroorganismen und Zellkulturen* (DSMZ) under deposit number DSM 34348; preferably, at a concentration comprised from $1\times10^6$ CFU to $1\times10^{12}$ CFU, more preferably comprised from $1\times10^8$ CFU to $1\times10^{10}$ CFU, e.g., $1\times10^9$ CFU.

3. The mixture according to claim 1 or 2, wherein said at least one strain of bacteria belonging to the species *Lactobacillus crispatus* is the strain *Lactobacillus crispatus* GA31 deposited at the *Deutsche Sammlung von Mikroorganismen und Zellkulturen* (DSMZ) under deposit number DSM 35105; preferably, at a concentration comprised from $1\times10^6$ CFU to $1\times10^{12}$ CFU, more preferably comprised from $1\times10^8$ CFU to $1\times10^{10}$ CFU, e.g., $1\times10^9$ CFU.

4. The mixture according to any one of claims 1-3, wherein:

   - said at least one strain of bacteria belonging to the species *Lactobacillus crispatus* is present in an amount by weight, with respect to the total weight of the mixture, preferably comprised in the range from 0.5% to 2.5%, more preferably comprised from 1% to 2%, even more preferably comprised from 1.3% to 1.9%, e.g., equal to 1.6%; and/or
   - said N-acetyl-L-cysteine (NAC) is present in an amount by weight, with respect to the total weight of the mixture, preferably comprised in the range from 20% to 50%, more preferably comprised from 27% to 45%, even more preferably comprised from 31% to 33%, e.g., equal to 32.15%; and/or

- said lycopene is present in an amount by weight, with respect to the total weight of the mixture, preferably comprised in the range from 1% to 3%, more preferably comprised from 1.5% to 2.5%, even more preferably comprised from 1.8% to 2%, e.g., equal to 1.9%.

5. The mixture according to any one of claims 1-4, wherein:

    - said vitamin D, preferably vitamin D3, is present in an amount by weight, with respect to the total weight of the mixture, preferably comprised in the range from 0.002% to 2%, more preferably comprised from 0.002% to 1.5%, even more preferably comprised from 0.002% to 1%, e.g., equal to 0.006%; and/or
    - said folic acid is present in an amount by weight, with respect to the total weight of the mixture, preferably comprised in the range from 20% to 90%, more preferably comprised from 40% to 80%, even more preferably comprised from 60% to 70%, e.g., equal to 64.3%.

6. The mixture according to any one of claims 1-5, wherein said mixture is for use as a medicament; preferably said mixture is for use as a medicament for oral use.

7. The mixture according to claim 6, wherein said mixture is for use in a method of treatment, preventive and/or curative, of female infertility, particularly to promote and enhance female gametogenesis, by promoting folliculogenesis, oocyte maturation, ovulation and the luteal phase, thus promoting the development of healthy mature gametes.

8. The mixture according to any one of claims 6 and 7, wherein said mixture is for use in a method, preventive and/or curative, to promote and enhance female gametogenesis, promote folliculogenesis, oocyte maturation, ovulation and luteal phase, stimulate cell viability and antioxidant mechanisms of ovarian cells, reduce the inflammatory state and apoptotic mechanisms of ovarian cells, promote the transition of meiosis from metaphase I to anaphase I in oocytes, maintain proper function of hormones deputed to gamete maturation, promote the development of healthy mature gametes, in subjects suffering from female infertility.

9. A composition comprising a mixture according to any one of claims 1-8 and, optionally, at least one acceptable pharmaceutical or food grade additive and/or excipient.

10. The composition according to claim 9, wherein said composition is for use as a medicament; preferably said composition is for use as a medicament for oral use.

11. The composition according to any one of claims 9 and 10, wherein said composition comprises:

    - an amount by weight of said at least one strain of bacteria belonging to the species *Lactobacillus crispatus* such as to provide a dosage from 5 mg to 20 mg, preferably from 7 mg to 13 mg, more preferably from 9 mg to 11 mg, even more preferably equal to 10 mg of *Lactobacillus crispatus;* and/or
    - an amount by weight of N-acetyl-L-cysteine (NAC) such as to provide a dosage from 100 mg to 300 mg, preferably from 150 mg to 250 mg, more preferably from 195 mg to 205 mg, even more preferably equal to 200 mg of N-acetyl-L-cysteine (NAC); and/or
    - an amount by weight of lycopene such as to provide a dosage from 5 mg to 20 mg, preferably from 7 mg to 14 mg, more preferably from 11 mg to 13 mg, even more preferably equal to 12 mg of lycopene; and/or
    - an amount by weight of vitamin D, preferably vitamin D3, such as to provide a dosage from 25 $\mu$g to 50 $\mu$g, preferably from 30 $\mu$g to 50 $\mu$g, more preferably from 35 $\mu$g to 45 $\mu$g, even more preferably equal to 40 $\mu$g of vitamin D; and/or
    - an amount by weight of folic acid (vitamin B9) such as to provide a total daily dosage from 250 mg to 500 mg, preferably from 300 mg to 500 mg, more preferably from 350 mg to 450 mg, even more preferably equal to 400 mg of folic acid.

12. The composition according to any one of claims 9-11, wherein said at least one strain of bacteria belonging to the species *Lactobacillus crispatus* is the strain *Lactobacillus crispatus* nova LCR deposited at the *Deutsche Sammlung von Mikroorganismen und Zellkulturen* (DSMZ) under deposit number DSM 34348; preferably, at a concentration comprised from $1\times10^6$ CFU to $1\times10^{12}$ CFU, more preferably comprised from $1\times10^8$ CFU to $1\times10^{10}$ CFU, e.g., $1\times10^9$ CFU; and/or the strain *Lactobacillus crispatus* GA31 deposited at the *Deutsche Sammlung von Mikroorganismen und Zellkulturen* (DSMZ) under deposit number DSM 35105; preferably, at a concentration comprised from $1\times10^6$ CFU to $1\times10^{12}$ CFU, more preferably comprised from $1\times10^8$ CFU to $1\times10^{10}$ CFU, e.g., $1\times10^9$ CFU.

13. The composition according to any one of claims 9-12, wherein said composition is for use in a method of treatment, preventive and/or curative, of female infertility, particularly to promote and enhance female gametogenesis, by promoting folliculogenesis, oocyte maturation, ovulation and luteal phase, promoting the development of healthy mature gametes.

14. The composition according to any one of claims 9-13, wherein said composition is for use in a method, preventive and/or curative, to promote and enhance female gametogenesis, promote folliculogenesis, oocyte maturation, ovulation and luteal phase, stimulate cell viability and antioxidant mechanisms of ovarian cells, reduce the inflammatory state and apoptotic mechanisms of ovarian cells, promote the transition of meiosis from metaphase I to anaphase I in oocytes, maintain proper function of hormones responsible for gamete maturation, promote the development of healthy mature gametes, in subjects suffering from female infertility.

**Figure 1**

Dose-response study of cell viability on CaCo-2 cells. The results of the MTT assay are expressed as mean ± SD (%) of 5 independent experiments, normalised versus the control (untreated cells). The highest curves show the best concentration for each substance tested.

**Figure 2**

Analysis on a 3D model of intestinal barrier of cell viability (at the top) carried out by means of the MTT assay and of ROS production (at the bottom) carried out via cytochrome C reduction. Data are expressed as mean ± SD (%) of 5 independent experiments, normalised versus the control (untreated cells). For all samples $p < 0.05$ vs control.

**Figure 3**

Analysis of transepithelial electrical resistance (TEER). Data are expressed as mean ± SD (%) of 5 independent experiments, each performed in triplicate and normalised versus the control values (%). The "breakpoint" corresponds to the control value (untreated cells).

**Figure 4**

Analysis of TJ activity (Claudin, Occludin, ZO-1). Data are expressed as mean ± SD (%) of 5 independent experiments, each performed in triplicate and normalised versus the control values (%). * p<0.05 vs control; # p<0.05 vs the single substances.

**Figure 5**

Analysis of the absorption on a 3D model of the intestinal barrier. Data are expressed as mean ± SD (%) of 5 independent experiments.

.

**Figure 6**

Evaluation of effectiveness in an *in vitro* model of the gut-ovary axis (viability, ROS production, SOD activity). Data are expressed as mean ± SD (%) of 5 independent experiments, each performed in triplicate and normalised versus the control values (%). * p<0.05 vs control; # p<0.05 vs single substances.

Figure 7

Evaluation of apoptotic activation in an *in vitro* model of the gut-ovary axis (ROS production, NO production). Data are expressed as mean ± SD (%) of 5 independent experiments, each performed in triplicate and normalised versus the control values (%). * p<0.05 vs control; # p<0.05 vs single substances.

**Figure 8**

Evaluation of apoptotic activation in an *in vitro* model of the gut-ovary axis (BAX activity, caspase 3 and cytochrome C). Data are expressed as mean ± SD (%) of 5 independent experiments, each performed in triplicate and normalised versus the control values (%). * $p < 0.05$ vs control; # $p < 0.05$ vs single substances.

**Figure 9**

Evaluation of anti-inflammatory activity in an *in vitro* model of the gut-ovary axis (COX-2 and PGE 2 activity). Data are expressed as mean ± SD (%) of 5 independent experiments, each performed in triplicate and normalised versus the control values (%). * $p < 0.05$ vs control; # $p < 0.05$ vs single substances.

Figure 10

Analysis of the main markers involved in the oocyte maturation process (CDK1, GDF-9 and BMP-15). Data are expressed as mean ± SD (%) of 5 independent experiments, each performed in triplicate and normalised versus the control values (%). * $p < 0.05$ vs control; # $p < 0.05$ vs single substances.

Figure 11

Analysis of progesterone and oestradiol levels. Data are expressed as mean ± SD (%) of 5 independent experiments, each performed in triplicate and normalised versus the control values (%). * p<0.05 vs control; # p<0.05 vs single substances.

Europäisches
Patentamt

European
Patent Office

Office européen
des brevets

# EUROPEAN SEARCH REPORT

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | KOORT KAIRI ET AL: "Lactobacillus crispatus-dominated vaginal microbiome and Acinetobacter-dominated seminal microbiome support beneficial ART outcome", ACTA OBSTETRICIA AND GYNECOLOGICA SCANDINAVICA., vol. 102, no. 7, 23 May 2023 (2023-05-23), pages 921-934, XP093149924, DK ISSN: 0001-6349, DOI: 10.1111/aogs.14598 * abstract * | 1-14 | INV. A61K35/742 A61K31/00 A61K31/19 A61K31/195 A61P15/08 |
| Y | TURKLER CAN ET AL: "An experimental study on the use of lycopene to prevent infertility due to acute oxidative ovarian damage caused by a single high dose of methotrexate", ADVANCES IN CLINICAL AND EXPERIMENTAL MEDICINE, vol. 29, no. 1, 21 January 2020 (2020-01-21), pages 5-11, XP093149823, PL ISSN: 1899-5276, DOI: 10.17219/acem/111809 * abstract * | 1-14 | TECHNICAL FIELDS SEARCHED (IPC) A61K A61P |
| Y | DATABASE MEDLINE [Online] US NATIONAL LIBRARY OF MEDICINE (NLM), BETHESDA, MD, US; 19 November 2020 (2020-11-19), DEVI NAGITA ET AL: "N-acetyl-cysteine as adjuvant therapy in female infertility: a systematic review and meta-analysis.", XP002811347, Database accession no. NLM34592079 * abstract * -/-- | 1-14 | |

The present search report has been drawn up for all claims

| Place of search | Date of completion of the search | Examiner |
|---|---|---|
| Munich | 11 February 2025 | Vandenbogaerde, Ann |

CATEGORY OF CITED DOCUMENTS

X : particularly relevant if taken alone
Y : particularly relevant if combined with another
document of the same category
A : technological background
O : non-written disclosure
P : intermediate document

T : theory or principle underlying the invention
E : earlier patent document, but published on, or
after the filing date
D : document cited in the application
L : document cited for other reasons

& : member of the same patent family, corresponding
document

EPO FORM 1503 03.82 (P04C01)

page 1 of 2

European
Patent Office

Europäisches
Patentamt

Office européen
des brevets

## EUROPEAN SEARCH REPORT

**Application Number**

EP 24 20 6491

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| | & DEVI NAGITA ET AL: "N-acetyl-cysteine as adjuvant therapy in female infertility: a systematic review and meta-analysis", JOURNAL OF BASIC AND CLINICAL PHYSIOLOGY AND PHARMACOLOGY, vol. 32, no. 5, September 2021 (2021-09), pages 899-910, ----- | | |
| Y | SCHAEFER ELLA ET AL: "The Impact of Preconceptional Multiple-Micronutrient Supplementation on Female Fertility", CLINICAL MEDICINE INSIGHTS. WOMEN'S HEALTH 2013, vol. 12, 1 January 2019 (2019-01-01), XP093149911, ISSN: 1179-562X, DOI: 10.1177/1179562X19843868 * the whole document * ----- | 1-14 | |

**TECHNICAL FIELDS SEARCHED (IPC)**

The present search report has been drawn up for all claims

| Place of search | Date of completion of the search | Examiner |
|---|---|---|
| Munich | 11 February 2025 | Vandenbogaerde, Ann |

CATEGORY OF CITED DOCUMENTS

X : particularly relevant if taken alone
Y : particularly relevant if combined with another
    document of the same category
A : technological background
O : non-written disclosure
P : intermediate document

T : theory or principle underlying the invention
E : earlier patent document, but published on, or
    after the filing date
D : document cited in the application
L : document cited for other reasons

& : member of the same patent family, corresponding
    document

EPO FORM 1503 03.82 (P04C01)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- *CHEMICAL ABSTRACTS*, 616-91-1
- *CHEMICAL ABSTRACTS*, 502-65-8
- *CHEMICAL ABSTRACTS*, 59-30-3